# EUROPÄISCHE PATENTSCHRIFT

(11) **EP 1 013 717 B1**
(45) Veröffentlichungstag und Bekanntmachung des Hinweises auf die Patenterteilung: **23.07.2003**
(21) Anmeldenummer: 99123980.7
(22) Anmeldetag: 07.12.1999
(51) Int. Cl.: C09B 29/085, C09B 29/033, C07C 309/65, C09B 29/039

(54) **Thermomigrierechte Azofarbstoffe**
Thermomigration-fast azo dyes
Colorants azoiques résistants à la thermomigration

(30) Priorität: 21.12.1998 DE 19858997
(43) Veröffentlichungstag der Anmeldung: 28.06.2000
(73) Patentinhaber: DyStar Textilfarben GmbH & Co. Deutschland KG, 65926 Frankfurt am Main (DE)
(72) Erfinder: Hamprecht, Rainer, Dr., 51519 Odenthal (DE)

(56) Entgegenhaltungen:
- EP-A- 0 313 918
- DE-A- 1 544 563
- DE-A- 2 916 861
- DE-A- 3 300 914
- DATABASE WPI Section Ch, Derwent Publications Ltd., London, GB; Class A60, AN 1972-76970T XP002134137 & JP 47 025488 A (MITSUI TOATSU CHEM INC)
- DATABASE WPI Section Ch, Derwent Publications Ltd., London, GB; Class A60, AN 1972-76969T XP002134138 & JP 47 025487 A (MITSUI TOATSU CHEM INC)

## Beschreibung

Gegenstand der Erfindung sind Azofarbstoffe der Formel 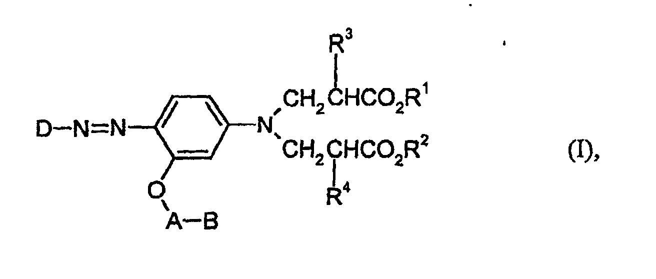 worin
D für einen aromatischen oder hetero-aromatischen Rest steht, wobei der Rest 2,4-Dinitro-6-ethansulfonylphenyl ausgenommen ist,
A für -CO- oder -SO₂-,
B für gegebenenfalls substituiertes C₁-C₄-Alkyl,
R¹ und R² unabhängig voneinander für gegebenenfalls durch OH oder C₁-C₄-Alkoxy substituiertes C₁-C₄-Alkyl und
R³ und R⁴ unabhängig voneinander für H oder CH₃ stehen,
mit der Bedingung, daß A für SO₂ steht, wenn D für einen 6-Nitro-Benzthiazolylrest steht.

Als Substituenten für das gegebenenfalls substituierte C₁-C₄-Alkyl in der Bedeutung von B kommen Cl, C₁-C₄-Alkoxy, C₁-C₄-Acyloxy, CO₂-C₁-C₄-Alkyl in Frage.

Geeignete aromatische Reste D sind substituierte Benzolderivate der Formel (II) 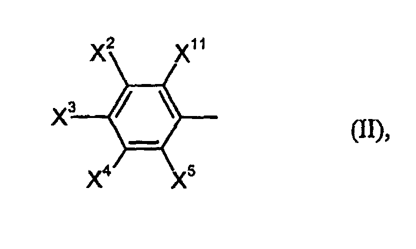 worin
X¹¹ und X⁵ unabhängig voneinander für H, F, Cl, Br, -NO₂, -CN, - CO₂-B, -OH, -CHO oder -CO-B stehen
X² und X⁴ unabhängig voneinander fiir H, Cl, Br, NO₂ oder B stehen,
X³ für H, Cl, Br, - NO₂, -CN, - SO₂-B, - CO₂-B, -CH=O oder B steht,
   und
B die oben angegebene Bedeutung hat.

Geeignete hetero-aromatische Reste D sind vorzugsweise Thiazole (IIIa), Isothiazole (IIIb), Thiophene (IIIc), Thiadiazole (IIId), Benzthiazole (IIIe) und Benzisothiazole (IIIf) der folgenden Formeln: 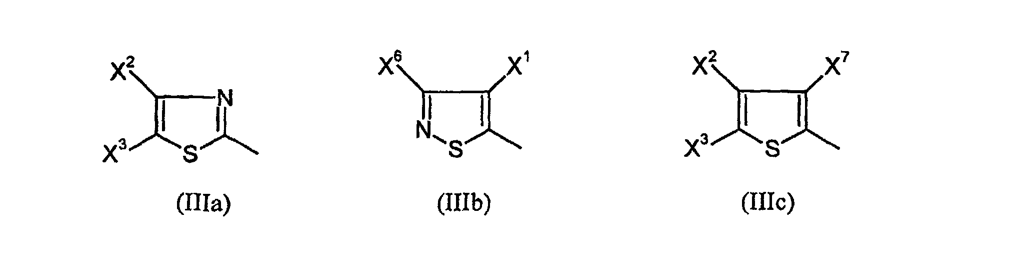 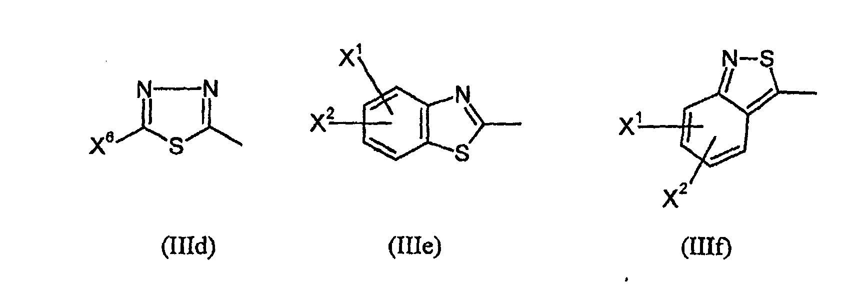 worin
X¹ für H; Cl, Br, CN, -NO₂, - SO₂-B oder CO₂-B steht und
X² bis X⁵ die obengenannte Bedeutung haben,
X⁶ für H, B, Cl, Br, -SB oder -SO₂B steht,
X⁷ für CN, -CO₂B oder -CO-B steht und
B die oben angegebene Bedeutung hat.

Bevorzugt sind Farbstoffe der Formel (I), worin
D für einen Rest der Formel 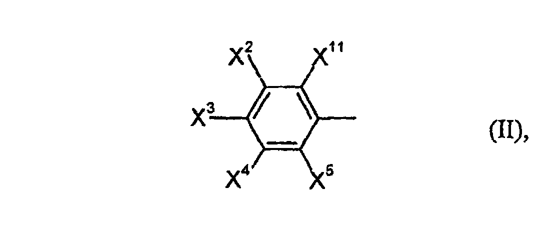 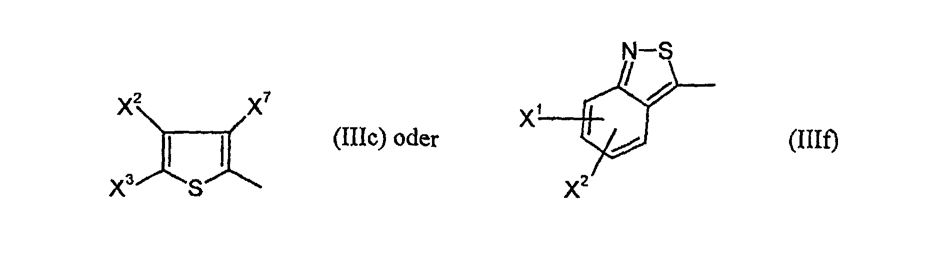 steht, worin
X¹¹ und X⁵ unabhängig voneinander für H, Cl, Br, CN, -NO₂, oder CO₂-B stehen,
X¹ für H, Cl, Br, CN, -NO₂, - SO₂-B oder CO₂-B steht
X² und X⁴ unabhängig voneinander für H, B, Cl oder Br stehen,
X³ fiir H, B, Cl, Br, -NO₂, CN, -CO₂-B oder -CH=O steht,
X⁷ für -CN, - CO₂-B, oder -CO-B steht und
B die oben angegebene Bedeutung hat.

Besonders bevorzugt sind Farbstoffe der Formel (I), worin
D für einen Rest der Formel 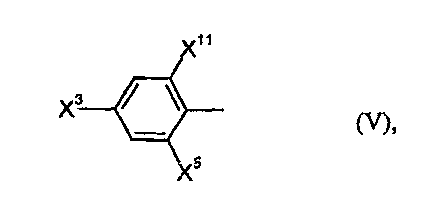 steht, worin
X¹¹ für H, Cl, Br, CN oder NO₂,
X³ für H, CH₃, Cl, Br oder NO₂,
X⁵ für H, Cl, Br oder CN steht,
A für -SO₂- steht,
B für gegebenenfalls durch Cl substituiertes C₁-C₄-Alkyl, insbesondere fiir Methyl steht,
R¹ und R² für C₁-C₄-Alkyl, insbesondere für Methyl stehen, und
R³ und R⁴ für Wasserstoff stehen.

Die Herstellung der Farbstoffe (I) erfolgt nach allgemein üblichen Methoden, beispielsweise indem man Diazokomponenten der Formel

D-NH₂, (VI)

worin
D die oben angegebene Bedeutung hat,
diazotiert und auf Kupplungskomponenten der Formel 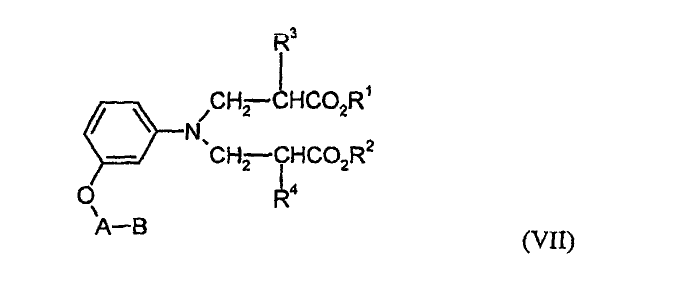 worin
A, B, R¹, R², R³ und R⁴ die oben angegebene Bedeutung haben
kuppelt.

Die Diazotierung erfolgt in üblicher Weise bei -20 bis 20°C mit Hilfe von Alkalinitriten, Nitrosylschwefelsäure oder Estern der salpetrigen Säure in Mineralsäuren (HCl, H₂SO₄, H₃PO₄) oder niederen Fettsäuren (Essig- und/oder Propionsäure). Auch die Kupplung wird nach üblichen Methoden durchgeführt, vorzugsweise durch Zugabe der wäßrigen Lösung oder Dispersion oder wäßrig/alkoholischen Lösung zur Diazoniumsalzlösung und gegebenenfalls Abstumpfen des Reaktionsgemisches auf pH 5-7 durch Zugabe von Alkali.

Ein bevorzugtes Verfahren zur Herstellung von erfindungsgemäßen 2,6-Dicyanazofarbstoffen der Formel 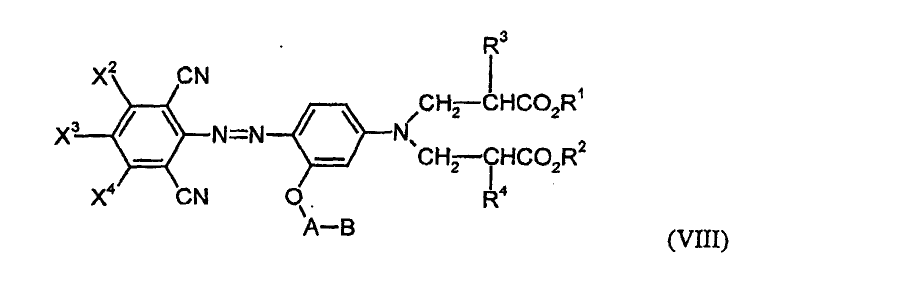 worin
R¹ bis R⁴, A, B und X² bis X⁴ die oben angegebene Bedeutung haben,
ist dadurch gekennzeichnet, daß man an nach an sich bekannten Verfahren hergestellten Azoverbindungen der Formel 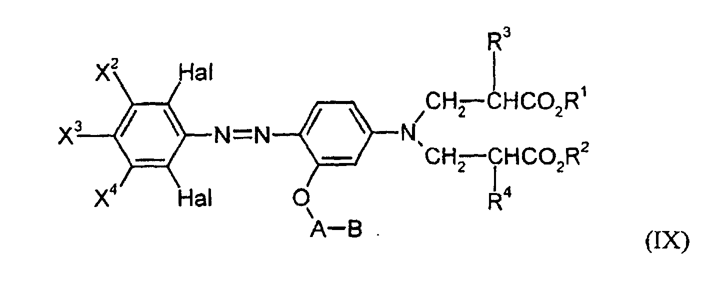 worin
R¹ bis R⁴, A, B und X² bis X⁴ die oben angegebene Bedeutung haben, und
Hal für Halogen, vorzugsweise Cl oder Br steht,
die Halogenatome gegen Cyangruppen austauscht.

Diese Austauschreaktion ist ebenfalls an sich bekannt und beispielsweise in folgender Patentliteratur beschrieben DE-A 1 544 563 = GB 1 125 683; DE-A 2 456 495 = GB 1 479 085; DE-A 2 759 103 = GB 2 012 799; DE-A 2 846 439 = GB 2 034 736; DD 217 232 (insbesondere die darin zitierte Literatur).

Danach wird die o,o'-Dihalogenazoverbindung in einem polaren organischen Lösungsmittel oder Wasser mit Metallcyaniden oder cyanidionenspendenden Verbindungen bei Temperaturen von 50 bis 150°C bis zum praktisch vollständigen Austausch der Halogenatome umgesetzt, was sich anhand von Dünnschichtchromatogrammen leicht verfolgen läßt.

Geeignete Cyanide sind vor allem CuCN und Zn(CN)₂ sowie komplexe Cyanide der Formel Me^{⊕}ₙ(CuCN)ₙ₊₁ (Me = Na, K; n = 1-3). Geeignete cyanidionenbildende Systeme sind z.B. Formaldoxim, Cyanhydrine, Nitroalkane oder Formamid.

Bevorzugte organische Lösungsmittel sind polare aprotische Verbindungen wie z.B. DMF, DMSO, Pyridin, N-Methylpyrrolidon, Chlorbenzol, Dichlorbenzole u.a.m.

Besonders geeignet und bevorzugt ist der Halogen/Cyan-Austausch mittels eines Gemisches von Kupfer(I)cyanid und Zinkcyanid.

Während die Diazokomponenten allgemein bekannt sind, sind von den KupplungsKomponenten nur die Vorläufer bekannt, vgl. z.B. EP 313 918 oder US 5 194 598.

Man erhält diese Verbindungen jedoch leicht in an sich bekannter Weise, z.B. durch Umsetzung von Verbindungen der Formel 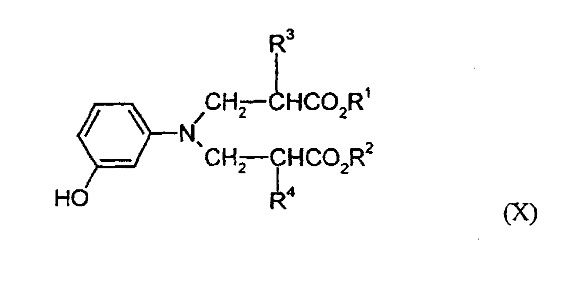 worin
R¹ bis R⁴ die oben angegebene Bedeutung haben
mit
a) Säureanhydriden, beispielsweise der Formel 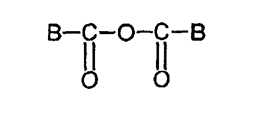 worin
   B die oben angegebene Bedeutung hat,
   oder
b) Säurechloriden der Formel

   B-A-Cl,

   worin
   B und A die oben angegebenen Bedeutungen haben.

Die Kupplungskomponenten der Formel (VII), worin B, R¹, R², R³ und R⁴ die unter Formel (I) fiir diese Substituenten angegebene Bedeutung haben, und A für SO₂ steht, sind neu und ebenfalls Gegenstand der vorliegenden Erfindung.

Diese Acylierungen werden vorzugsweise in organischen, vorzugsweise wasserfreien Lösungsmitteln durchgeführt. Auch Zweiphasensysteme Wasser/organisches Lösungsmittel sind geeignet.

Geeignete Lösungsmittel sind beispielsweise unpolare organische Lösungsmittel wie chlorierte Kohlenwasserstoffe, beispielsweise Methylenchlorid, Tetrachlorkohlenstoff, aromatische Lösungsmittel, beispielsweise Toluol, Xylole, Chlorbenzol, Dichlorbenzol, Nitrobenzol, daneben sind auch polare Lösungsmittel wie Aceton, Dimethylformamid, N-Methylpyrrolidon, Sulfolan usw. geeignet.

Zuweilen ist das Abfangen des entstehenden Chlorwasserstoffs mit anorganischen oder organischen Basen vorteilhaft.

Geeignete Basen sind beispielsweise Natriumhydroxid, Kaliumhydroxid, Soda, Pottasche, Natrium- oder Kaliumhydrogencarbonat, Natriumhydrogenphosphat, Natriumacetat sowie Amine wie z.B. Trialkylamine, Pyridin, Dialkylaminopyridin, Chinolin, Dialkylaniline usw.

Die erfindungsgemäßen Farbstoffe sind, wie für Dispersionsfarbstoffe üblich, in Wasser praktisch unlöslich. Sie eignen sich vorzugsweise zum Färben und Bedrucken von synthetischen Textilmaterialien, insbesondere Textilmaterialien aus sekundärem Celluloseacetat und Cellulosetriacetat, Polyamid, wie z.B. Polyhexamethylenadipamid, und ganz besonders aromatischem Polyester, wie z.B. Polyethylenterephthalat nach den für diese Faserarten üblichen Färbe- und Druckmethoden unter Verwendung wäßriger oder nicht wäßriger Flotten oder Druckpasten.

Die Farbstoffe können schließlich auch auf synthetische Textilmaterialien durch Thermotransferdruck aufgebracht werden.

Weiterhin eignen sich die Farbstoffe für den thermischen Transferdruck auf synthetischen Aufzeichnungsmaterialen in optischen Aufzeichnungsverfahren, z.B. zum Bedrucken von polyesterbeschichtetem Papier.

Die mit den neuen Farbstoffen erhaltenen scharlachfarbigen bis blauen Färbungen auf Polyester zeichnen sich bei guten Allgemeinechtheiten durch hohe Farbstärke, klare Farbtöne und insbesondere hervorragende Thermomigrierechtheit aus.

Gegenüber nächstvergleichbaren bekannten Farbstoffen gemäß JP-A 47 25 487 die eine 6-Nitro-benzthiazol Diazokomponente und JP-A 4725488, die eine 2,4-Dinitro-6-ethylsulfonyl-phenyl Diazokomponente aufweisen, zeichnen sich die neuen Farbstoffe durch verbesserte Echtheitseigenschaften aus.

### Beispiele

### Beispiel 1

Herstellung des Farbstoffs der Formel

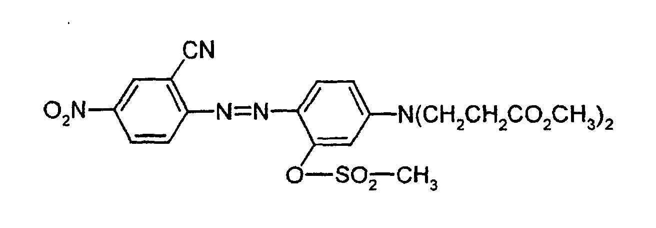

Zu einer Lösung von 7,3 g 2-Cyan-4-nitroanilin in 30 ml Propionsäure und 60 ml Eisessig tropfte man bei 0 bis 5°C in 30 Minuten unter Rühren 8,3 ml einer 42-prozentigen Nitrosylschwefelsäurelösung in Schwefelsäure zu. Zwei Stunden wurde bei 0°C nachgerührt.

Zu einer Lösung von 16,4 g β-Alanin, N-(3-methoxy-3-oxopropyl)-N-[3-[(methansulfonyl)oxy]phenyl]-methylester und 2 g Amidosulfonsäure in 200 ml Methanol ließ man bei 0°C obige Diazotierung langsam zulaufen, wobei durch Zusatz von Eiswasser auf ein Volumen von 1 500 ml aufgefüllt wurde. Nach Rühren über Nacht wurde abgesaugt und neutral gewaschen. Ausbeute: 19,9 g. Das Rohprodukt ließ sich aus DMF umkristallisieren.
λₘₐₓ: 503 nm (CH₂Cl₂)

Der Farbstoff färbt Polyester in einem blaustichigen Rot mit guten Echtheiten, vor allem sehr guter Thermomigrierechtheit.

Die benötigte Kupplungskomponente läßt sich durch Umsetzung von β-Alanin, N-(3-methoxy-3-oxopropyl)-N-[3-[oxy]phenyl]-methylester mit Methansulfochlorid in Toluol bei 70°C in Gegenwart von Triethylamin gewinnen.

Nach analogen oder ähnlichen Verfahren lassen sich die in den nachfolgenden Tabellen angegebenen Farbstoffe der Formel 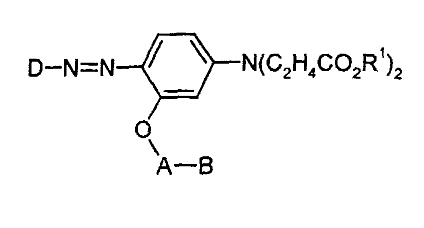 herstellen, wobei in Tabelle 1 Farbstoffe mit A = SO₂ und in Tabelle 2 Farbstoffe mit A = CO beschrieben sind.

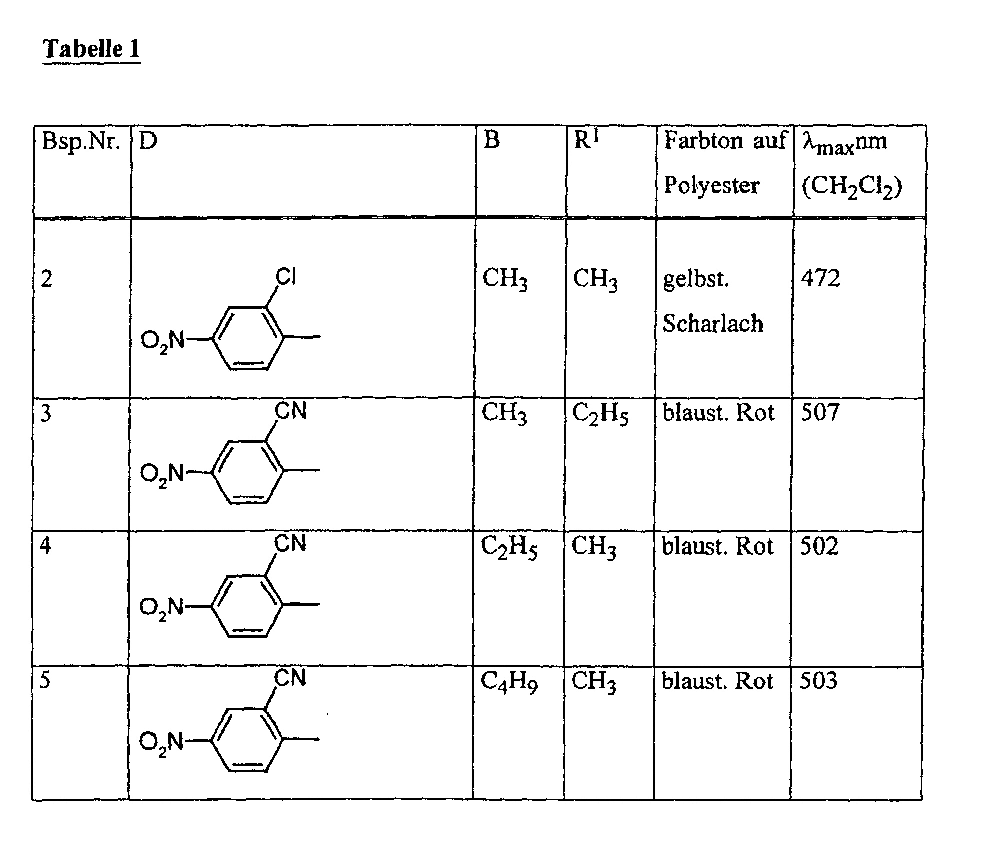

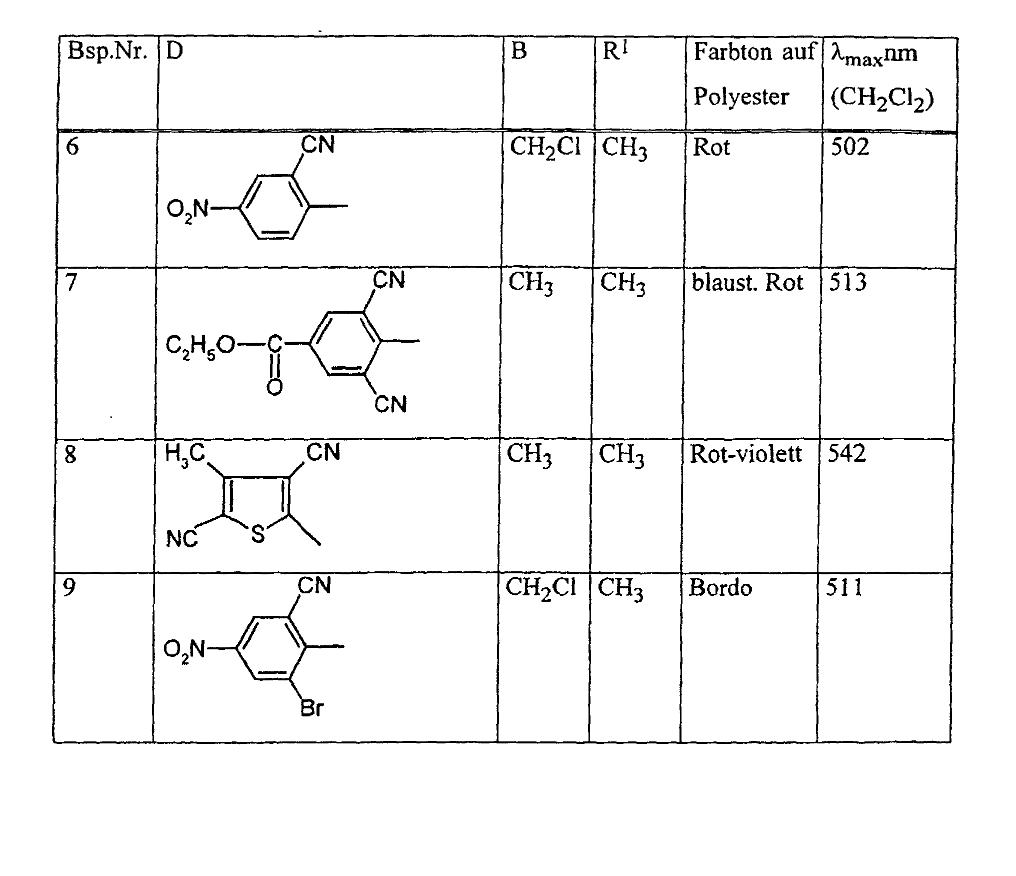

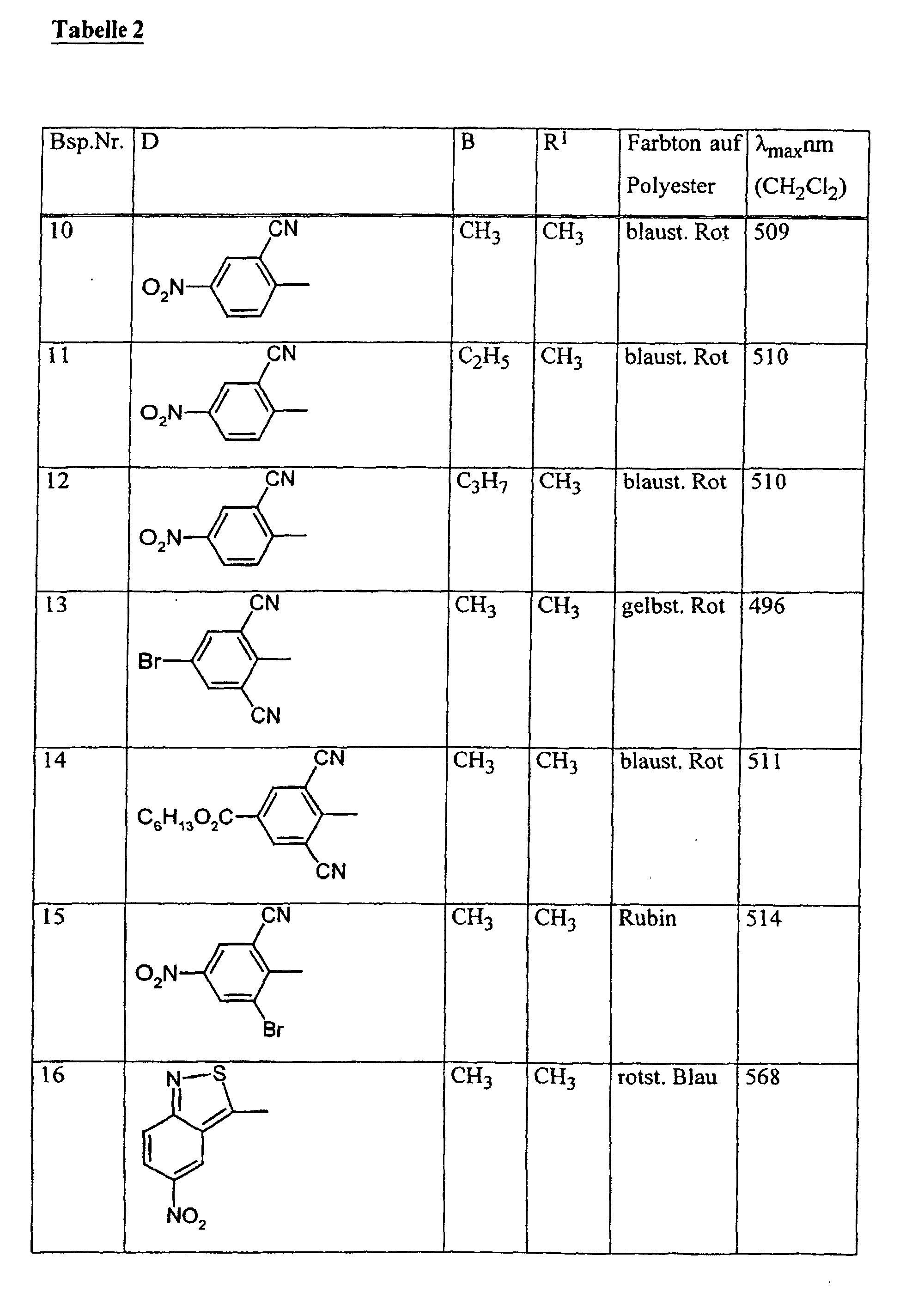

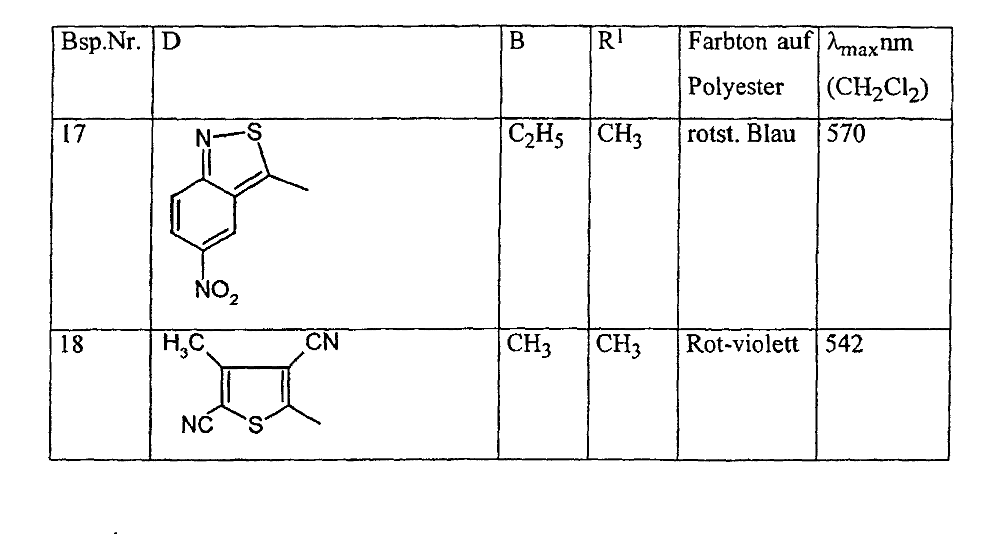

## Patentansprüche

1. Farbstoffe der Formel 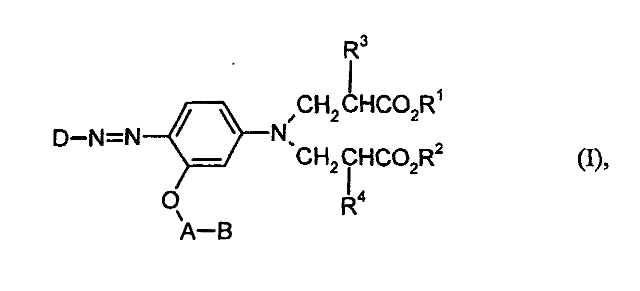 worin
D für einen aromatischen oder hetero-aromatischen Rest steht, wobei der Rest 2,4-Dinitro-6-ethansulfonylphenyl ausgenommen ist,
A für -CO- oder -SO₂-,
B für gegebenenfalls substituiertes C₁-C₄-Alkyl,
R¹ und R² unabhängig voneinander fiir gegebenenfalls durch OH oder C₁-C₄-Alkoxy substituiertes C₁-C₄-Alkyl und
R³ und R⁴ unabhängig voneinander für H oder CH₃ stehen,
mit der Bedingung, daß A für SO₂ steht, wenn D fiir einen 6-Nitro-Benzthiazolylrest steht.

2. Farbstoffe gemäß Anspruch 1, worin
D für einen Rest der Formel 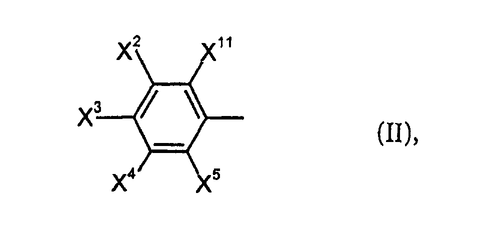 steht, worin
X¹¹ und X⁵ unabhängig voneinander für H, F, Cl, Br, -NO₂, -CN, -CO₂-B, -OH, -CHO oder -CO-B stehen,
X² und X⁴ unabhängig voneinander für H, Cl, Br, NO₂ oder B stehen,
X³ für H, Cl, Br, - NO₂, -CN, - SO₂-B, - CO₂-B, -CH=O oder B steht,
und
B die in Anspruch 1 angegebene Bedeutung hat.

3. Farbstoffe gemäß Anspruch 1, worin
D fiir einen Rest der Formeln (IIIa) bis (IIIf) steht 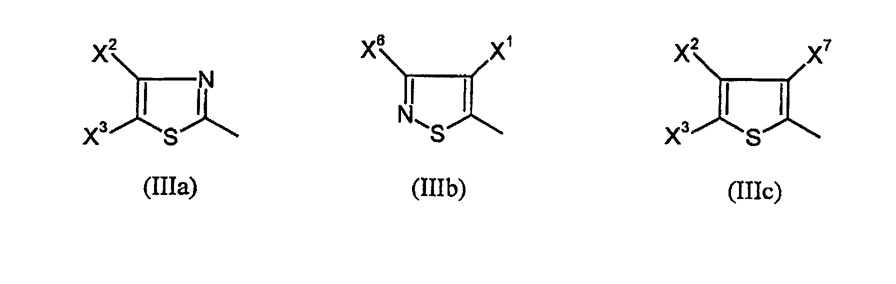 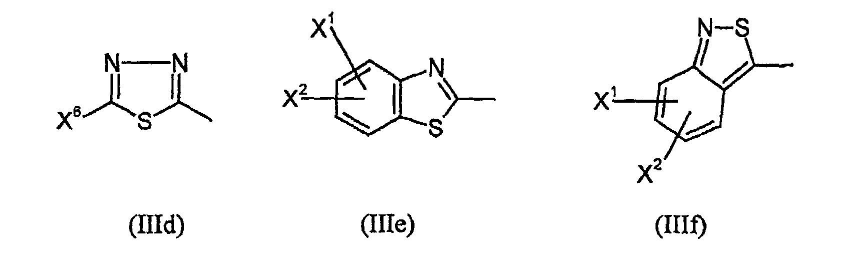 worin
X¹ für H, Cl, Br, CN, -NO₂, - SO₂-B oder CO₂-B steht,
X² bis X⁵ die in Anspruch 2 angegebene Bedeutung haben,
X⁶ für H, B, Cl, Br, -SB oder -SO₂B steht,
X⁷ für CN, -CO₂B oder -CO-B und
B die in Anspruch 1 angegebene Bedeutung hat.

4. Farbstoffe gemäß Ansprüchen 1 bis 3, worin
D für einen Rest der Formel 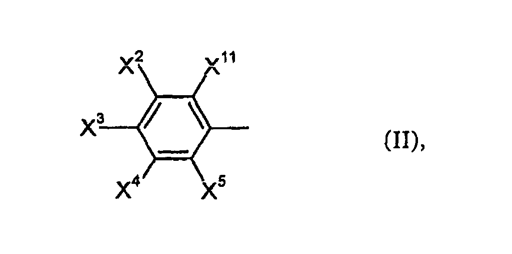 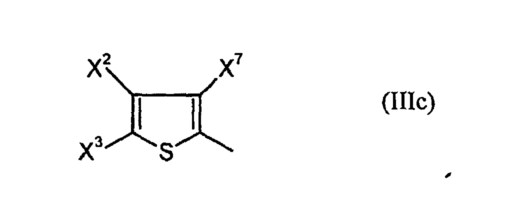 oder 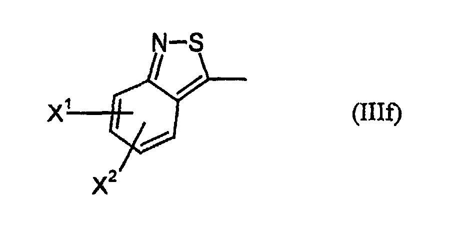 steht, worin
X¹¹ und X⁵ unabhängig voneinander für H, Cl, Br, CN, -NO₂, oder CO₂-B stehen,
X¹ für H, Cl, Br, CN, -NO₂, - SO₂-B oder CO₂-B steht
X² und X⁴ unabhängig voneinander für H, B, Cl oder Br stehen,
X³ für H, B, Cl, Br, -NO₂, CN, -CO₂-B oder -CH=O steht,
X⁷ für -CN, - CO₂-B, -CO-B steht und
B die in Anspruch 1 angegebene Bedeutung hat,

5. Farbstoffe gemäß Anspruch 1, worin
D für einen Rest der Formel 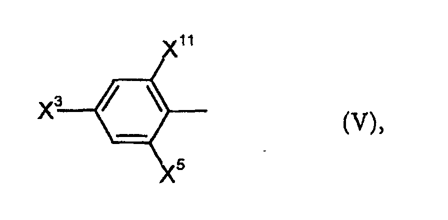 steht, worin
X¹¹ für H, Cl, Br, CN oder NO₂,
X³ für H, CH₃, Cl, Br oder NO₂,
X⁵ für H, Cl, Br oder CN steht,
A für -SO₂- steht,
B für gegebenenfalls durch Cl substituiertes C₁-C₄-Alkyl, insbesondere für Methyl steht,
R¹ und R² für C₁-C₄-Alkyl, insbesondere für Methyl stehen, und
R³ und R⁴ für Wasserstoff stehen.

6. Farbstoffe gemäß Anspruch 1 der Formel 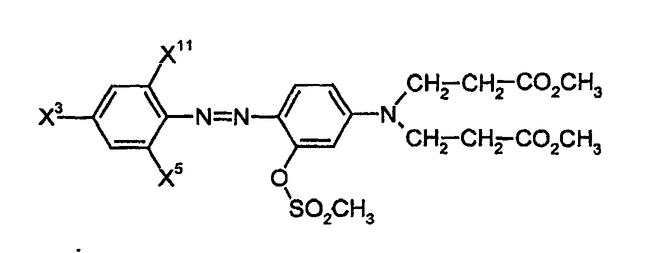 worin
X¹¹, X³ und X⁵ die in Anspruch 5 angegebene Bedeutung haben.

7. Verfahren zur Herstellung von Farbstoffen gemäß Ansprüchen 1 bis 6, dadurch gekenzeichnet, daß man Diazokomponenten der Formel
D-NH₂ (VI)
worin
D die in Anspruch 1 angegebene Bedeutung hat,
diazotiert und auf Kupplungskomponenten der Formel 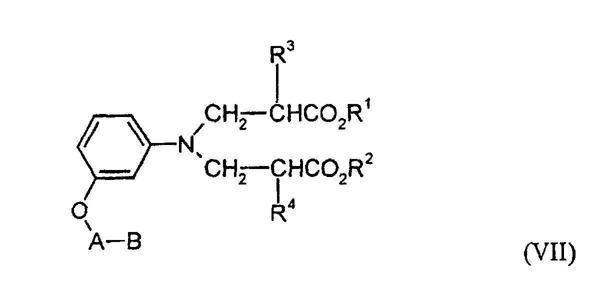
worin
A, B, R¹, R², R³ und R⁴ die in Anspruch 1 angegebene Bedeutung haben, kuppelt.

8. Verbindungen der Formel 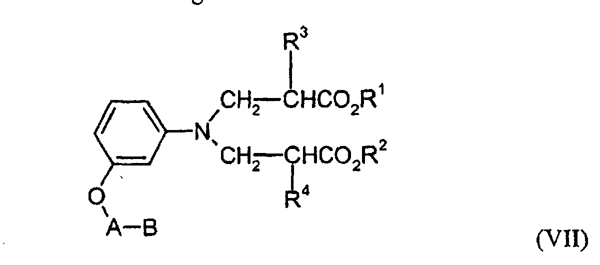 worin
B, R¹, R², R³ und R⁴ die in Anspruch 1 angegebene Bedeutung haben, und A für SO₂ steht.

9. Verwendung von Farbstoffen gemäß Anspruch 1 bis 6, zum Färben und Bedrucken von synthetischen Textilmaterialien, insbesondere von sekundärem Celluloseacetat, Cellulosetriacetat, Polyamid und aromatischem Polyester.

10. Verwendung von Farbstoffen gemäß Anspruch 1 bis 6, für den Thermotransferdruck auf synthetischen Textilmaterialien, oder auf synthetischen Aufzeichnungsmaterialien in optischen Aufzeichnungsverfahren.

## Claims

1. Dyes of the formula 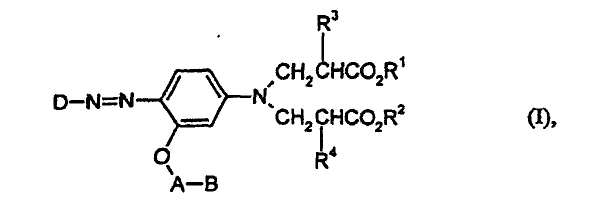 wherein
D is aryl or hetaryl other than 2,4-dinitro-6-ethanesulphonylphenyl,
A is -CO- or -SO₂-,
B is optionally substituted C₁-C₄-alkyl,
R¹ and R² are independently optionally OH- or C₁-C₄-alkoxy-substituted C₁-C₄-alkyl, and
R³ and R⁴ are independently H or CH₃,
with the proviso that A is SO₂ when D is a 6-nitrobenzothiazolyl radical.

2. Dyes according to Claim 1, wherein
D is a radical of the formula 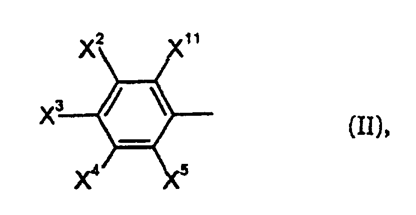 wherein
X¹¹ and X⁵ are independently H, F, Cl, Br, -NO₂, -CN, -CO₂-B, -OH, -CHO or -CO-B,
X² and X⁴ are independently H, Cl, Br, NO₂ or B,
X³ is H, Cl, Br, -NO₂, -CN, -SO₂-B, -CO₂-B, -CH=O or B,
and
B is as defined in Claim 1.

3. Dyes according to Claim 1, wherein
D is a radical of the formulae (IIIa) to (IIIf) 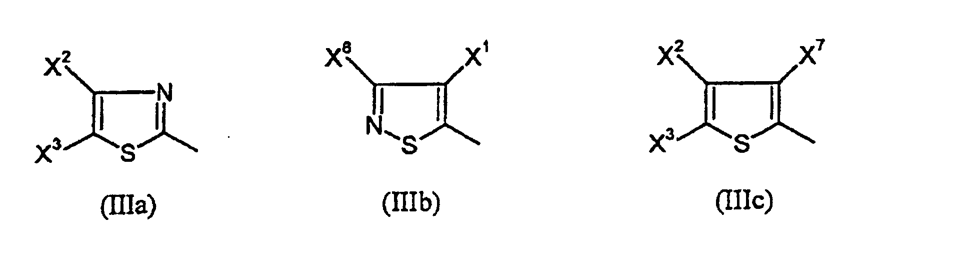 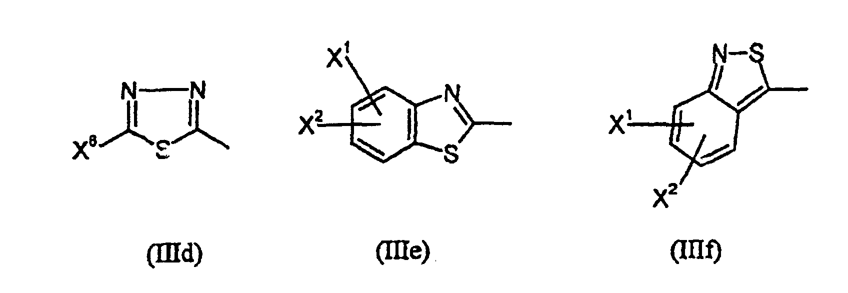 wherein
X¹ is H, Cl, Br, CN, -NO₂, -SO₂-B or CO₂-B,
X² to X⁵ are each as defined in Claim 2,
X⁶ is H, B, Cl, Br, -SB or -SO₂B,
X⁷ is CN, -CO₂B or -CO-B, and
B is as defined in Claim 1.

4. Dyes according to Claims 1 to 3, wherein
D is a radical of the formula 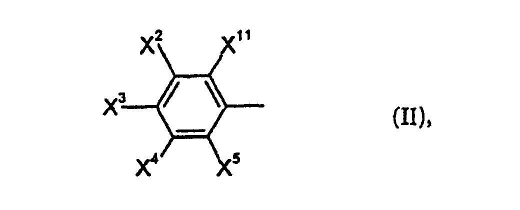 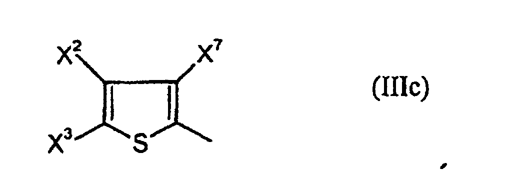 or 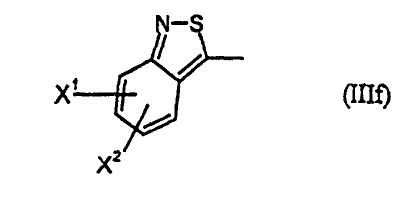 wherein
X¹¹ and X⁵ are independently H, Cl, Br, CN, -NO₂, or CO₂-B,
X¹ is H, Cl, Br, CN, -NO₂, -SO₂-B or CO₂-B,
X² and X⁴ are independently H, B, Cl or Br,
X³ is H, B, Cl, Br, -NO₂, CN, -CO₂-B or -CH=O,
X⁷ is -CN, -CO₂-B, -CO-B, and
B is as defined in Claim 1.

5. Dyes according to Claim 1, wherein
D is a radical of the formula 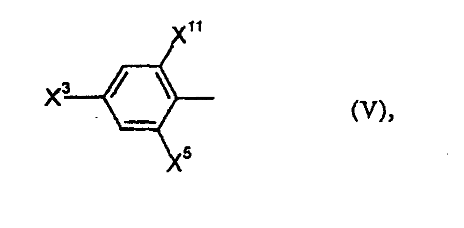 wherein
X¹¹ is H, Cl, Br, CN or NO₂,
X³ is H, CH₃, Cl, Br or NO₂,
X⁵ is H, Cl, Br or CN,
A is -SO₂-,
B is optionally Cl-substituted C₁-C₄-alkyl, especially methyl,
R¹ and R² are each C₁-C₄-alkyl, especially methyl, and
R³ and R⁴ are each hydrogen.

6. Dyes according to Claim 1 of the formula 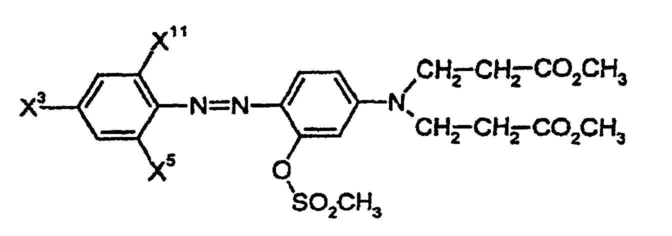 wherein
X¹¹, X³ and X⁵ are each as defined in Claim 5.

7. Process for preparing dyes according to Claims 1 to 6, **characterized in that** diazo components of the formula
D-NH₂ (VI)
wherein
D is as defined in Claim 1,
are diazotized and coupled onto coupling components of the formula 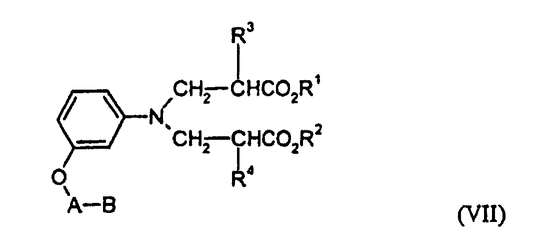 wherein
A, B, R¹, R², R³ and R⁴ are each as defined in Claim 1.

8. Compounds of the formula 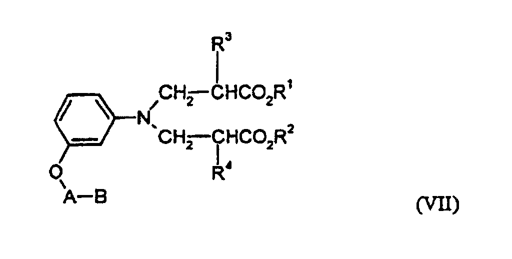 wherein
B, R¹, R², R³ and R⁴ are each as defined in Claim 1 and A is SO₂.

9. Use of dyes according to Claim 1 to 6 for dyeing and printing synthetic textile materials especially secondary cellulose acetate, cellulose triacetate, polyamide and aromatic polyester.

10. Use of dyes according to Claim 1 to 6 for thermal transfer printing on synthetic textile materials or on synthetic recording materials in optical recording processes.

## Revendications

1. Colorants de formule 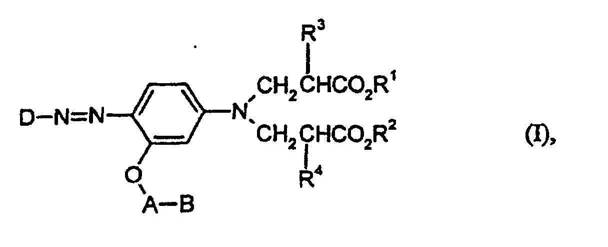 dans laquelle
D représente un radical aromatique ou hétéro-aromatique, à l'exclusion du radical 2,4-dinitro-6-éthanesulfonylphényle,
A représente -CO- ou -SO₂-,
B représente un groupe alkyle en C₁-C₄ éventuellement substitué,
R¹ et R² représentent, indépendamment l'un de l'autre, un radical alkyle en C₁-C₄ éventuellement substitué par OH ou un groupe alcoxy en C₁-C₄ et
R³ et R⁴ représentent, indépendamment l'un de l'autre, H ou CH₃,
étant entendu que A représente SO₂ lorsque D représente un radical 6-nitro-benzothiazolyle.

2. Colorants selon la revendication 1, dans lesquels
D représente un radical de formule 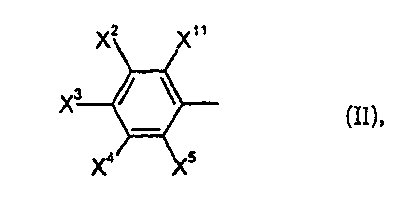 dans laquelle
X¹¹ et X⁵ représentent, indépendamment l'un de l'autre, H, F, Cl, Br, -NO₂, -CN, -CO₂-B, -OH, -CHO ou -CO-B,
X² et X⁴ représentent, indépendamment l'un de l'autre, H, Cl, Br, NO₂ ou B,
X³ représente H, Cl, Br, -NO₂, -CN, -SO₂-B, -CO₂-B, -CH=O ou B,
et
B a la signification donnée dans la revendication 1.

3. Colorants selon la revendication 1, dans lesquels
D représente un radical de formules (IIIa) à (IIIf) 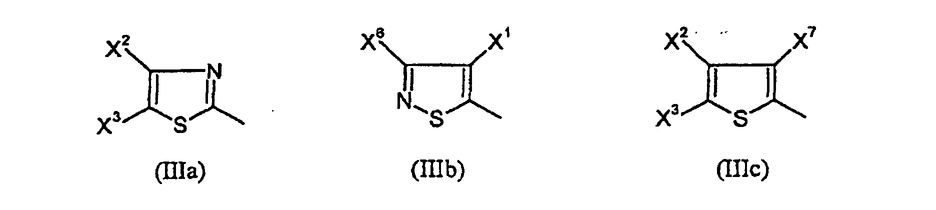 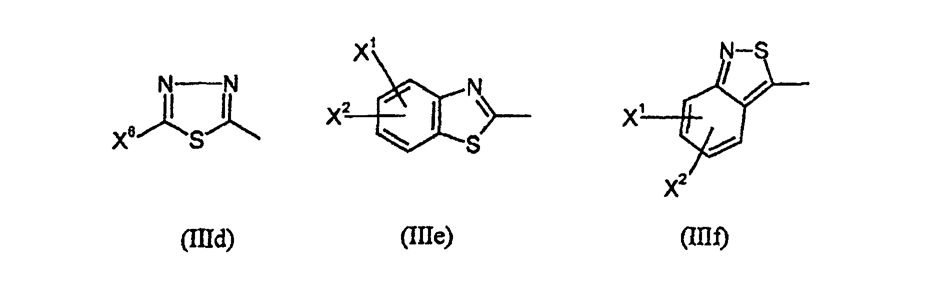 dans lesquelles
X¹ représente H, Cl, Br, CN, -NO₂, SO₂-B, ou CO₂-B,
X² à X⁵ ont les significations données dans la revendication 2,
X⁶ représente H, B, Cl, Br, -SB ou -SO₂B,
X⁷ représente CN, -CO₂B ou -CO-B et
B a la signification donnée dans la revendication 1.

4. Colorants selon les revendications 1 à 3, dans lesquels
D représente un radical de formule 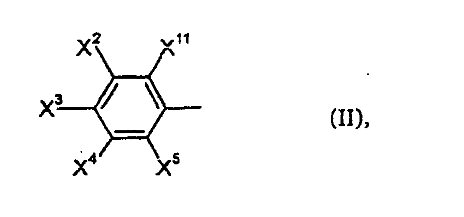 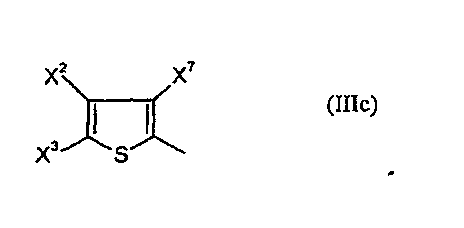 ou 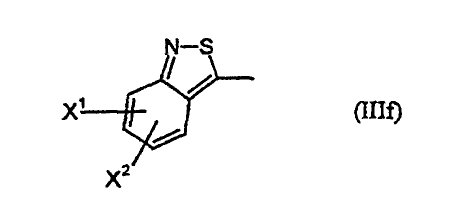 formules dans lesquelles
X¹¹ et X⁵ représentent, indépendamment l'un de l'autre, H, Cl, Br, CN, -NO₂ ou CO₂-B,
X¹ représente H, Cl, Br, CN, -NO₂, -SO₂-B, ou CO₂-B,
X² à X⁴ représentent, indépendamment l'un de l'autre, H, B, Cl ou Br,
X³ représente H, B, Cl, Br, -NO₂, CN, -CO₂-B ou -CH=O,
X⁷ représente -CN, -CO₂-B ou -CO-B et
B a la signification donnée dans la revendication 1.

5. Colorants selon la revendication 1, dans lesquels
D représente un radical de formule 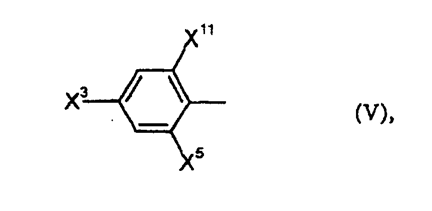 dans laquelle
X¹¹ représente H, Cl, Br, CN ou NO₂,
X³ représente H, CH₃, Cl, Br ou NO₂,
X⁵ représente H, Cl, Br ou CN,
A représente -SO₂-,
B représente un radical alkyle en C₁-C₄, éventuellement substitué par Cl, en particulier le groupe méthyle,
R¹ et R² représentent un groupe alkyle en C₁-C₄, en particulier le groupe méthyle, et
R³ et R⁴ représentent des atomes d'hydrogène.

6. Colorants selon la revendication 1, de formule 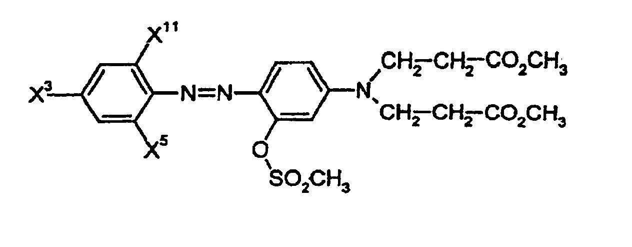 dans laquelle
X¹¹, X³ et X⁵ ont les significations données dans la revendication 5.

7. Procédé pour la préparation des colorants selon les revendications 1 à 6, **caractérisé en ce qu'**on soumet à une diazotation des composants diazotables de formule
D-NH₂ (VI)
dans laquelle
D a la signification donnée dans la revendication 1, et on les fait copuler sur des copulants de formule 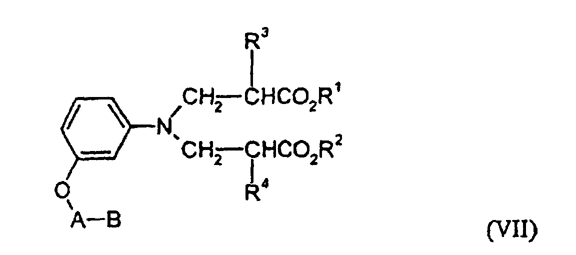 dans laquelle
A, B, R¹, R², R³ et R⁴ ont les significations données dans la revendication 1.

8. Composés de formule 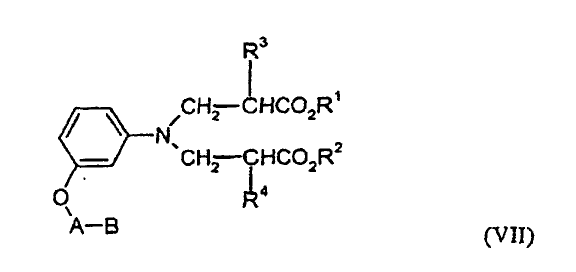 dans laquelle
B, R¹, R², R³ et R⁴ ont les significations données dans la revendication 1 et A représente SO₂.

9. Utilisation des colorants selon les revendications 1 à 6, pour la teinture et l'impression de matériaux textiles synthétiques, en particulier d'acétate de cellulose secondaire, triacétate de cellulose, polyamide et polyester aromatique.

10. Utilisation des colorants selon les revendications 1 à 6, pour l'impression par transfert thermique sur des matériaux textiles synthétiques ou sur des matériaux d'enregistrement synthétiques dans des procédés d'enregistrement optique.
